(19) Europäisches Patentamt
European Patent Office
Office européen des brevets

(11) EP 1 261 575 B1

(12) # FASCICULE DE BREVET EUROPEEN

(45) Date de publication et mention de la délivrance du brevet:
**29.12.2004 Bulletin 2004/53**

(21) Numéro de dépôt: **00968006.7**

(22) Date de dépôt: **11.10.2000**

(51) Int Cl.$^7$: **C07C 51/567**, C07C 57/04

(86) Numéro de dépôt international:
**PCT/FR2000/002826**

(87) Numéro de publication internationale:
**WO 2001/028969 (26.04.2001 Gazette 2001/17)**

(54) **PROCEDE PERFECTIONNE DE FABRICATION D'ANHYDRIDES SYMETRIQUES**

VERFAHREN ZUR HERSTELLUNG VON SYMMETRISCHEN ANHYDRIDEN

IMPROVED METHOD FOR PRODUCING SYMMETRICAL ANHYDRIDES

(84) Etats contractants désignés:
**DE ES FR GB**

(30) Priorité: **19.10.1999 FR 9913015**

(43) Date de publication de la demande:
**04.12.2002 Bulletin 2002/49**

(73) Titulaire: **Atofina**
**92800 Puteaux (FR)**

(72) Inventeurs:
- **PAUL, Jean-Michel**
  **F-57070 Metz (FR)**
- **FABIS, Frédéric**
  **F-14920 Mathieu (FR)**
- **RAULT, Sylvain**
  **F-14370 Moult (FR)**

(56) Documents cités:
**FR-A- 2 592 040** **FR-A- 2 774 375**

- **S. KIM: "A simple and mild esterification method for carboxylic acids using mixed carboxylic-carbonic anhydrides" JOURNAL OF ORGANIC CHEMISTRY, vol. 50, no. 5, 1985, pages 560-565, XP002141427 EASTON US cité dans la demande**
- **J. S. NELSON: "Symmetrical anhydrides of hydroxy acids" JOURNAL OF ORGANIC CHEMISTRY, vol. 28, juillet 1963 (1963-07), pages 1905-1907, XP002141428 EASTON US cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention porte sur un procédé de fabrication d'anhydrides symétriques représentés par la formule générale (I) :

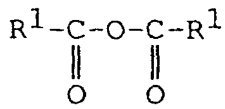

(I)

dans laquelle $R^1$ représente un groupement alkyle, alcényle, aryle, alkaryle ou aralkyle.

Dans la présente description, on désignera les anhydrides de la forme :

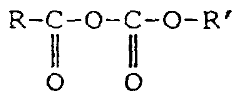

R et R' représentant des groupements hydrocarbonés sous le terme générique "anhydride mixte" en précisant pour ceux de la forme :

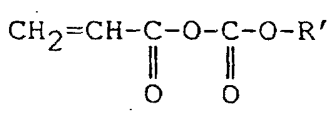

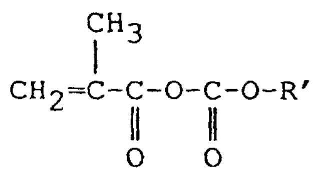

"anhydride mixte respectivement acrylique et méthacrylique".

Les anhydrides symétriques sont des agents d'acylation doux utilisables dans la synthèse d'esters, d'amides, de thioesters, ou en tant qu'agents de réticulation (dans le cas, par exemple, des anhydrides (méth)acryliques).

Les procédés de synthèse des anhydrides symétriques décrits dans la littérature sont les suivants :

- déshydratation d'acides carboxyliques en présence de catalyseurs ;
- réaction d'acides carboxyliques ou de leurs sels avec des chlorures d'acides ou des composés chlorés tels que $SOCl_2$, $SO_2Cl_2$, $PCl_5$, $PCl_3$, $POCl_3$, etc. ; et
- réaction d'un acide carboxylique avec l'anhydride acétique en présence ou non d'un catalyseur tel que $H_2SO_4$ et $ZnCl_2$.

La synthèse des anhydrides (méth)acryliques à partir d'acide (méth)acrylique et d'anhydride acétique est en particulier décrite dans la demande de brevet français FR-A-2 592 040.

La synthèse d'anhydrides symétriques non acryliques à partir d'anhydrides mixtes a été notamment décrite dans la littérature par Jane S. Nelson, L.A. Goldblatt et T.H. Applewhite in J. Org. Chem. 1963, 28, 1905 et par Sunggak Kim, Jae In Lee et Youn Chul Kim in J. Org. Chem. 1985, 50, 560-565.

Les conditions de synthèse mettant en jeu notamment une basse température, un milieu solvant tel que $CH_2Cl_2$, tétrahydrofuranne, etc., et l'utilisation de triéthylamine en grande quantité, rendent cependant cette voie peu envisageable industriellement en tant que telle et économiquement inintéressante comparativement à celle décrite dans la demande de brevet français FR-A-2 592 040.

La synthèse d'anhydrides symétriques à partir d'anhydride acétique est assez délicate en série (méth)acrylique. On se heurte en effet à des problèmes de polymérisation, à la formation de résidus lourds, à la gestion de fractions intermédiaires à recycler, etc.

Il importait donc de mettre au point un nouveau procédé simplifié de synthèse d'anhydrides symétriques qui ne présente pas les défauts du procédé classiquement utilisé tel que décrit dans les articles précités de Nelson et al. et Kim et al., et qui pallie les difficultés indiquées ci-dessus, particulièrement en série (méth)acrylique.

La présente invention a donc pour objet un procédé de fabrication d'un anhydride symétrique, représenté par la formule générale (I) :

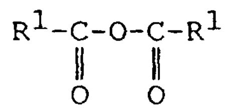

(I)

dans laquelle $R^1$ représente un groupement alkyle, alcényle, aryle, alkaryle ou aralkyle,
caractérisé par le fait que l'on fait réagir un anhydride mixte de formule (II) :

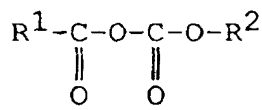

(II)

dans laquelle :

- $R^1$ est tel que défini ci-dessus ; et
- $R^2$ représente un reste alkyle en $C_1$-$C_{40}$, alcényle en $C_1$-$C_{40}$, aryle, aryl-(alkyle en $C_1$-$C_{40}$) et (alkyl en $C_1$-$C_{40}$)-aryle,

avec un acide carboxylique de formule (III) :

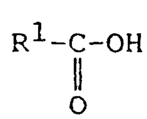

(III)

dans laquelle $R^1$ est tel que défini ci-dessus.

Le groupement alkyle entrant dans la définition de $R^1$ est notamment un groupe alkyle en $C_1$-$C_{40}$, linéaire, ramifié ou cyclique, tel qu'octyle ; le groupement alcényle entrant dans la définition de $R^1$ est notamment un groupe alcényle en $C_2$-$C_{40}$, tel que vinyle, isopropényle ; le groupement aryle entrant dans la définition de $R^1$- est notamment un groupe phényle ; le groupement alkaryle entrant dans la définition de $R^1$ est notamment un groupe tolyle ; le groupement aralkyle entrant dans la définition de $R^1$ est notamment un groupe benzyle.

De façon préférentielle $R^1$ représente un reste vinyle $H_2C=CH-$ ou isopropényle

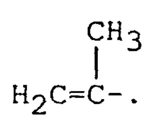

Parmi les restes alkyle en $C_1$-$C_{40}$ entrant dans la définition de $R^2$, on peut citer les restes méthyle, éthyle, n-propyle, isopropyle, n-butyle et isobutyle, ceux-ci étant d'un intérêt particulier. A titre d'exemples de restes alcényle, aryle, aralkyle et alkaryle entrant dans la définition de $R^2$, on peut citer les mêmes que ceux donnés ci-dessus pour $R^1$.

Conformément à un premier mode de réalisation du procédé selon la présente invention, on introduit l'anhydride mixte (II) sous forme pure ou sous la forme du produit de réaction d'un carboxylate alcalin de formule générale (IV) :

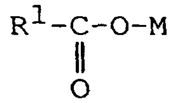

(IV)

dans laquelle :

- $R^1$ est tel que défini ci-dessus, et
- M est un métal alcalin, tel que Na, K,

avec un chloroformiate de formule générale (V) :

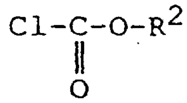

(V)

dans laquelle $R^2$ est tel que défini ci-dessus.

En particulier, on peut introduire le produit de réaction du carboxylate alcalin (IV) et du chloroformiate (V), ladite réaction ayant été conduite en milieu aqueux, le carboxylate alcalin (IV) ayant pu être préparé en solution aqueuse par neutralisation de l'acide carboxylique correspondant par l'hydroxyde MOH, avant sa réaction avec le chloroformiate (V), et le produit de réaction brut ayant pu être purifié en particulier pour éliminer l'excès de chloroformiate (V).

La préparation des anhydrides mixtes (méth)acryliques (II) pour lesquels $R^1$ représente $H_2C=CH-$ et $H_2C=C(CH)_3-$, est décrite dans la demande de brevet français FR-A-2 774 375 au nom de la Société déposante, et dont le contenu est incorporé par référence à la présente description. Les conditions réactionnelles décrites dans cette demande de brevet sont extrapolables à la préparation des autres anhydrides mixtes de formule (II).

Dans ce premier mode de réalisation de la présente invention, la réaction entre l'anhydride mixte (II) et l'acide carboxylique (III) qui conduit à l'anhydride symétrique (I) est la suivante :

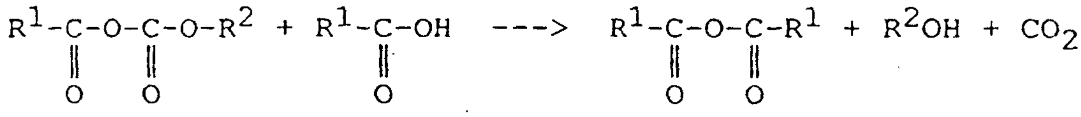

Le rapport molaire acide carboxylique (III)/anhydride mixte (II) est dans ce cas avantageusement compris entre 1 et 3, de préférence entre 1 et 1,2.

La réaction conforme à ce premier mode de réalisation est généralement conduite à une température de -10 à 70°C, de préférence de 0 à 20°C.

Par ailleurs, la réaction entre l'anhydride mixte (II) et l'acide carboxylique (III) est avantageusement conduite en présence d'un catalyseur choisi notamment parmi les carbonates alcalins ou alcalino-terreux, tels que $K_2CO_3$ et $Na_2CO_3$, les résines acides ou basiques, telles que la résine Amberlist 15/A26/IRA400, les substances minérales telles que les zéolithes et le silicate de magnésium (Florisil®), et les sels de Zn, Mn et Mg, tels que MgO, $MgSO_4$, $MgCl_2$. L'utilisation d'un tel catalyseur permet d'accélérer la synthèse.

On introduit le catalyseur à raison notamment d'au moins 0,0001 mole par mole d'anhydride mixte (II), de préférence à raison de 0,001 à 0,01 mole par mole d'anhydride mixte (II).

Un catalyseur préféré est l'oxyde de magnésium. Conformément à un second mode de réalisation du procédé selon l'invention, on prépare l'anhydride mixte (II) in situ. L'anhydride symétrique (I) est alors obtenu directement, sans isoler l'anhydride mixte (II), par réaction d'un chloroformiate de formule (V) :

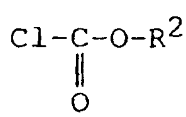

(V)

dans laquelle $R^2$ est tel que défini ci-dessus, avec un carboxylate alcalin de formule générale (IV) :

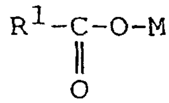 (IV)

ou un mélange carboxylate alcalin de formule (IV)/acide carboxylique (III) :

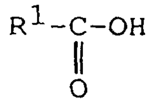 (III)

dans lesquelles :

- $R^1$ est tel que défini ci-dessus, et
- M est un métal alcalin, tel que Na, K.

Le rapport molaire carboxylate alcalin (IV) + acide carboxylique (III)/ chloroformiate (V) est dans ce cas avantageusement compris entre 1 et 3, de préférence entre 1,5 et 1,9.

La réaction conforme à ce second mode de réalisation est conduite à une température généralement de -10 à 70°C, de préférence de 0 à 40°C.

Par ailleurs, la réaction entre le carboxylate alcalin de formule générale (IV) ou le mélange carboxylate alcalin (IV) / acide carboxylique (III) et le chloroformiate de formule (V) est avantageusement conduite en présence d'un catalyseur de transfert de phase, dissous ou fixé sur un support polymérique et utilisé notamment à raison de 0,001 à 0,02 mole, en particulier de 0,005 à 0,01 mole, par mole de carboxylate alcalin (IV).

Le support polymérique peut être un copolymère styrène-divinylbenzène ou une résine polyvinylpyridine réticulée.

Le catalyseur de transfert de phase est avantageusement choisi parmi les sels d'ammonium quaternaires, les sels de phosphonium et les éthers-couronne.

A titre d'exemples de sels d'ammonium Quaternaires

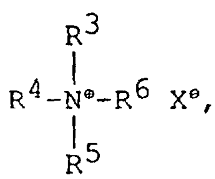

on peut citer ceux dans lesquels :

- $R^3$ à $R^6$ représentent chacun alkyle en $C_1$-$C_{40}$, tel que $CH_3$, $C_2H_5$, $C_4H_9$, $C_8H_{17}$, $C_{16}H_{33}$, ou aryle tel que phényle, ou aralkyle tel que benzyle ;
- X représente l'un parmi Cl, Br, I, OH et $HSO_4$ ;

et, en particulier, le chlorure de tétraméthylammonium, le chlorure de benzyltriméthylammonium, le chlorure de benzyltri-n-butylammonium, le chlorure de tétra-n-butyl ammonium, le bromure de tétra-n-butylammonium, le bromure de méthyltrioctylammonium et l'hydrogénosulfate de tétra-n-butylammonium.

A titre d'exemples de sels de phosphonium, on peut citer $(C_4H_9)_4P^{\oplus}Cl^{\oplus}$, $(C_4H_9)_4P^{\oplus}Br^{\oplus}$, $(C_8H_{17})_3C_2H_5P^{\oplus}Cl^{\oplus}$, $(C_4H_9)_3C_6H_5P^{\oplus}Cl^{\oplus}$.

A titre d'exemples d'éthers couronne, on peut citer le 18-crown-6 et le dibenzoyl-18-crown-6.

Dans les premier et second modes de réalisation précités, on introduit généralement dans le milieu réactionnel au moins un inhibiteur de polymérisation, à raison de 500 à 5000 ppm, en particulier de 500 à 1000 ppm, par rapport à l'anhydride mixte (II) ou au carboxylate alcalin (IV) de départ lorsque ces derniers comportent une double liaison

engendrant des risques de polymérisation (cas de l'anhydride mixte (méth)acrylique (II) et du (méth)acrylate alcalin (IV)). L'inhibiteur de polymérisation, qui permet alors de surstabiliser ces composés comportant des doubles liaisons, est avantageusement choisi parmi l'éther monométhylique de l'hydroquinone, l'hydroquinone, la phénothiazine et le ditertiobutylhydroxytoluène (ditertio-butylparacrésol).

Le procédé selon la présente invention, qui peut s'envisager en batch ou en continu, permet de fabriquer les anhydrides symétriques de formule (I) dans de meilleures conditions de productivité, et, dans le cas des anhydrides (méth)acryliques, avec beaucoup moins de risques de polymérisation que par la voie partant de l'acide acétique,

Les Exemples suivants illustrent la présente invention sans toutefois en limiter la portée. Dans ces exemples, les pourcentages sont en poids sauf indication contraire, et les abréviations utilisées sont les suivantes :

Réactifs et Produits

AMA : acide méthacrylique
AA : acide acrylique
$AMA_2O$ : anhydride méthacrylique
$AA_2O$ : anhydride acrylique
MAE : méthacrylate d'éthyle
Anhydride mixte méthacrylique-Et :

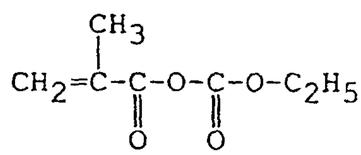

Inhibiteur de polymérisation

BHT : ditertiobutylhydroxytoluène
EMHQ : éther monométhylique de l'hydroquinone

EXEMPLE 1

Dans un réacteur à double enveloppe, agité mécaniquement, surmonté d'une colonne à distiller pouvant fonctionner sous dépression, équipée d'un condenseur, d'une recette et d'un piège, on introduit 196,4 g d'une solution d'anhydride mixte méthacrylique brute contenant

- 72,4 % d'anhydride mixte méthacrylique-Et

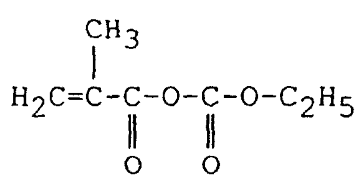

- 25,2 % d'$AMA_2O$,
- 3,9 % de $ClCOOC_2H_5$, et
- 0,9 % de MAE.

On ajoute 78 g d'AMA (rapport molaire anhydride mixte méthacrylique / AMA = 1).

On ajoute ensuite 0,02 g d'oxyde de magnésium MgO et 2000 ppm (0,55 g)de BHT.

Le mélange est agité à température ambiante (circulation d'eau à 20°C dans la double enveloppe) pendant

30 minutes. Un dégagement de $CO_2$ est observé.

Le brut ainsi préparé qui ne contient plus d'anhydride mixte méthacrylique-Et, lequel a été entièrement converti en $AMA_2O$, est ensuite purifié par distillation.

Les constituants légers sont éliminés par étêtage à une pression initiale de 2 x $10^4$ Pa (150 mmHg) et finale de 2,67 x $10^3$ Pa (20 mmHg).

Une fraction de tête de 77 g ayant la composition suivante :

| | |
|---|---|
| $ClCOOC_2H_5$ | 5,3 %, |
| MAE | 26, 4 %, |
| $CO(OC_2H_5)_2$ | 2,8 %, |
| AMA | 22,8 %, |
| éthanol | 39,5%, |
| $AMA_2O$ | 1,5 % |

est ainsi distillée.

Le résidu de distillation (150 g) est constitué à 97 % par de l'$AMA_2O$.

EXEMPLES 2 à 6

Un mélange équimolaire d'anhydride mixte méthacrylique-Et (composition massique = 95 % d'anhydride mixte méthacrylique-Et, 5 % d'$AMA_2O$) et d'AMA est agité dans un bécher à température ambiante en présence de catalyseur.

Un dégagement de $CO_2$ se produit lors de la réaction, ce qui entraîne une chute de la température du milieu réactionnel de 20 à 5°C.

L'anhydride mixte méthacrylique-Et est entièrement converti avec une excellente sélectivité en $AMA_2O$ lorsqu'on opère à faible teneur en catalyseur.

Les résultats sont rapportés dans le Tableau 1 ci-après.

Tableau 1

| Exemple | 2 | 3 | 4 | 5 | 6 |
|---|---|---|---|---|---|
| Composition finale | Nature du catalyseur : MgO | | | Nature du catalyseur : Silicate de magnésium (Florisil®) | |
| Quantité de catalyseur | 0,1 mole/mole d'anhydride mixte | 0,01 mole/mole d'anhydride mixte | 0,0005 mole/mole d'anhydride mixte | 1 mole/mole d'anhydride mixte | 0,1 mole/mole d'anhydride mixte |
| Durée de réaction | < 1 min | 20 min | 1 h | > 1 h | > 5 h |
| $AMA_2O$ % | 33 | 88 | 86 | 78 | 83 |
| AMA % | 33 | 10 | 12 | 16 | 16 |
| MAE % | 33 | <2 | < 2 | 6 | < 2 |

EXEMPLE 7

Dans un bécher contenant 15 g d'anhydride mixte méthacrylique-Et (composition massique : 87 % d'anhydride mixte méthacrylique-Et, 13 % d'$AMA_2O$), on ajoute à température ambiante un équivalent d'AMA par rapport à l'anhydride mixte, soit 7,1 g, puis 0,0005 éq de MgO, soit 2 mg.

On observe un dégazage et, après une heure de réaction, la composition massique du milieu est la suivante : > 95 % en $AMA_2O$ et < 5 % en MAE. Il ne reste plus d'AMA détectable par RMN.

EXEMPLES 8 à 15

Différents anhydrides mixtes de formule brute générale

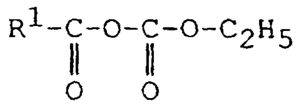

sont mis en réaction avec leur acide

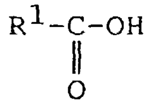

à la température de 20°C en présence de 0,01 mole de MgO par rapport à l'anhydride mixte.

Le rapport molaire acide/anhydride mixte = 1.

On observe un dégagement de $CO_2$. Lorsque celui-ci a cessé, on lave le brut avec de l'eau. On récupère la phase organique par décantation. Après séchage sur $MgSO_4$, celle-ci est analysée par $RMN^1H$.

Les résultats obtenus sont regroupés dans le Tableau 2 ci-après :

Tableau 2

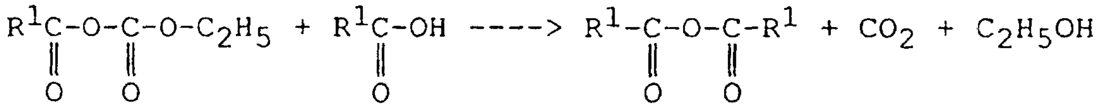

| Exemple | 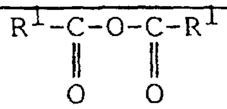 | Rendement % (par rapport à l'anhydride mixte) |
|---|---|---|
| 8 | 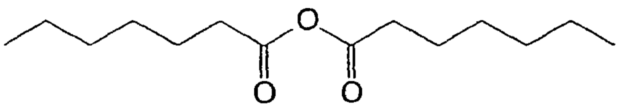 | 85 |
| 9 | 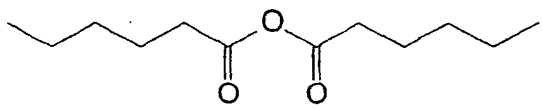 | 95 |
| 10 | 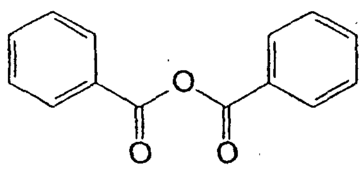 | 92 |
| 11 | 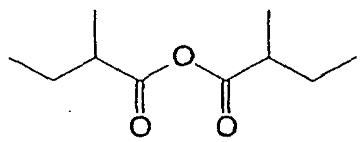 | 87 |
| 12 | 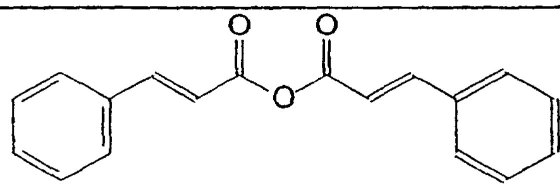 | 90 |
| 13 | 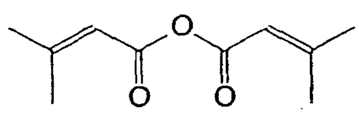 | 83 |
| 14 | 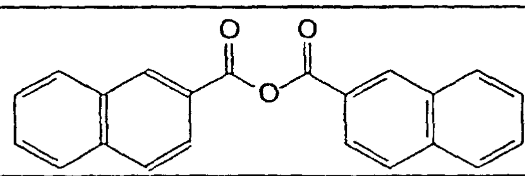 | 95 |
| 15 | 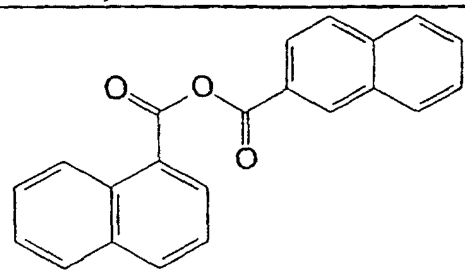 | 95 |

EXEMPLE 16

Dans le réacteur de l'Exemple 1, on charge 577,4 g de solution aqueuse de soude à 20,6 %. On introduit en continu, à 25°C maximum, 255,8 g d'acide méthacrylique stabilisé avec 2000 ppm d'EMHQ.
La vitesse d'introduction de l'AMA est ajustée en fonction de la température dans le réacteur. En fin d'introduction, on rajoute 130,6 g d'eau, afin d'avoir un rapport massique eau/méthacrylate de sodium = 2.
On introduit ensuite, en une fois, 333,1 g de chloroformiate d'éthyle et 0,24 g de bromure de tétrabutylammonium.
Le mélange est laissé en réaction, sous agitation, à 40°C pendant 4 h, puis décanté à température ambiante.
La phase aqueuse est éliminée. La phase organique (404 g) contient :

- 5,5 % de $ClCOOC_2H_5$ ;
- 0,2 % de MAE ;
- 0,05% d'AMA ;
- 70,2 % d'anhydride mixte méthàcrylique-Et ; et
- 24 % d'$AMA_2O$.

On y ajoute 154,4 g d'AMA et on laisse sous agitation à température ambiante pendant 1 h. Le brut est ensuite réchauffé sous vide (pression de 2 x $10^4$ Pa (150 mmHg) ) et étêté (distillation des produits légers). Un dégagement de $CO_2$ est observé durant la phase d'agitation initiale à température ambiante et en début de phase d'étêtage.
En l'absence de MgO, la conversion de l'anhydride mixte en $AMA_2O$ (dont le dégagement de $CO_2$ est un traceur) est plus lente qu'en présence de catalyseur.
L'étêtage du brut est effectué en baissant progressivement la pression de service de 2 x $10^4$ à 2,67 x $10^3$ Pa (de 150 à 20 mmHg).
La composition finale du brut étêté est la suivante :

| | |
|---|---|
| $AMA_2O$ | 99,1 % ; |
| Ethanol | 0,03 % ; |
| MAE | 0, 1 %; |
| $CO(OC_2H_5)_2$ | 0,03 %. |

EXEMPLE 17

Dans le réacteur de l'Exemple 1, on charge 480 g de solution aqueuse de soude à 20,0 %. On introduit en continu, à 25°C maximum, 358,4 g d'acide méthacrylique stabilisé avec 2000 ppm d'EMHQ.
La vitesse d'introduction de l'AMA est ajustée en fonction de la température dans le réacteur.
Dans 830 g de cette solution, on introduit en une fois 260 g de chloroformiate d'éthyle et 0,73 g de bromure de tétrabutylammonium.
Le mélange est laissé en réaction, sous agitation, à 20°C pendant 4 h, puis décanté à température ambiante.
La phase organique (416 g) est maintenue sous agitation à 40°C pendant 1 heure supplémentaire. Elle est ensuite étêtée sous vide. Après élimination des légers (éthanol, MAE, AMA, $CO(OEt)_2$, eau), on récupère 277 g d'$AMA_2O$ en pied de colonne d'une pureté > 98%.

EXEMPLE 18

L'Exemple 17 est repris en remplaçant le chloroformiate d'éthyle par du chloroformiate de méthyle.
Dans le réacteur de l'Exemple 1, on charge 500 g de solution aqueuse de soude à 20,9 %. On introduit, dans les mêmes conditions qu'à l'Exemple 17, 430,2 g d'AMA.
Dans la solution de méthacrylate de sodium et d'acide méthacrylique ainsi préparée, on ajoute en une fois 266,3 g de chloroformiate de méthyle et 0,83 g de bromure de tétrabutylammonium.
Le mélange est laissé en réaction, sous agitation, pendant 1,5 heure à 20°C, puis décanté à température ambiante.
La phase organique (496 g) est maintenue à 40°C pendant 1 heure supplémentaire, puis étêtée sous vide. On récupère ainsi 260 g de produit de pied de colonne constitué à plus de 98% par de l'$AMA_2O$.

EXEMPLES 19 à 26

La synthèse d'anhydrides symétriques

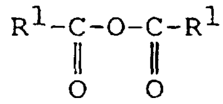

est effectuée en neutralisant l'acide carboxylique

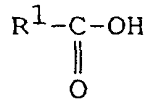

avec 1,05 éq. de soude 5N à une température < 25°C, puis en faisant réagir, in situ, le sel de sodium avec le chloroformiate d'éthyle (0,55 éq. par rapport à l'acide) pendant 2 h à température ambiante. On récupère la phase organique par décantation. Après lavage à l'eau puis séchage sur $MgSO_4$, on l'analyse par RMN$^1$H.

Les résultats obtenus sont regroupés dans le Tableau 3 ci-après :

Tableau 3

| Exemple | 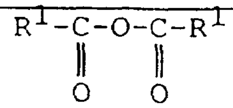 | Rendement % (par rapport à l'anhydride mixte) |
|---|---|---|
| 19 | 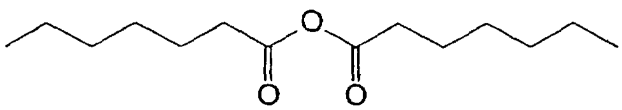 | 92 |
| 20 | 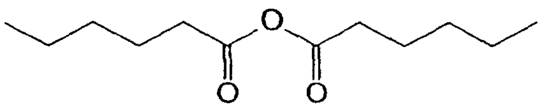 | 80 |
| 21 | 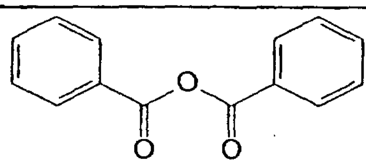 | 64 |
| 22 | 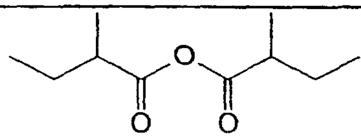 | 75 |
| 23 | 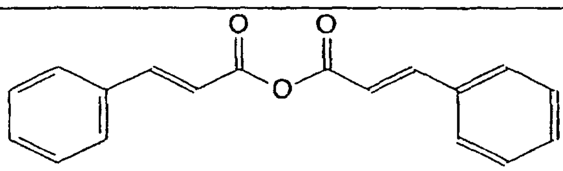 | 96 |
| 24 | 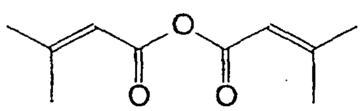 | 60 |
| 25 | 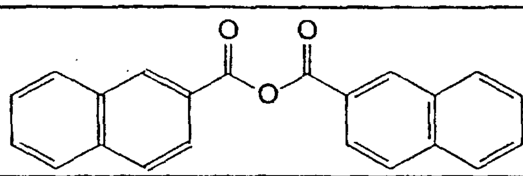 | 60 |
| 26 | 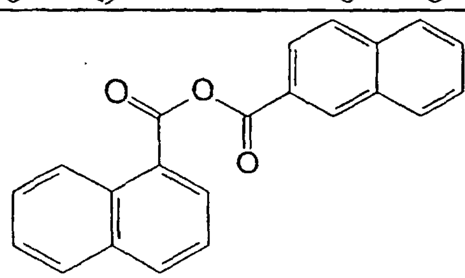 | 90 |

EXEMPLE 27

Dans un réacteur refroidi à 0°C, agité à l'aide d'un barreau magnétique contenant 123 ml de solution de potasse 5N et surmonté d'un réfrigérant, on introduit goutte à goutte 42,8 g d'AA stabilisé avec 1000 ppm d'EMHQ

(1,05 éq KOH/mole AA). On ajoute ensuite 60 ml d'eau et on laisse sous agitation à 15°C pendant 1 heure.

On additionne ensuite en une fois 30 ml de chloroformiate d'éthyle (0,52 éq., soit 0,31 mole) et 1 % de bromure de tétrabutylammonium. On laisse l'ensemble sous agitation pendant 2,5 h à 15°C jusqu'à conversion complète de l'acrylate de potassium.

Le brut réactionnel décante en deux phases à température ambiante.

L'$AA_2O$ présent dans la phase organique est obtenu avec un rendement de 85 %.

<u>EXEMPLE 28</u>

Dans le réacteur de l'Exemple 18, on introduit 123 ml d'une solution aqueuse de potasse 5N, et on ajoute goutte à goutte 42,9 g d'AA contenant 1000 ppm d'EMHQ en maintenant la température du réacteur à 0°C.

On ajoute ensuite 60 ml d'eau et on laisse sous agitation pendant 1 h supplémentaire à température ambiante.

On introduit ensuite en une fois 74,9 g de chloroformiate d'éthyle et 1 % de bromure de tétrabutylammonium. On laisse sous agitation à 15°C jusqu'à conversion complète de l'acrylate de potassium (1,5 h). La phase organique est décantée. La composition massique du mélange est de 70 % d'anhydride mixte acrylique et 30 % d'$AA_2O$(RMN$^1$H). L'excès de chloroformiate d'éthyle est éliminé sous vide (2,67 x $10^3$ Pa (20 mmHg)) à l'évaporateur rotatif.

On ajoute 19,3 g d'AA et 50 mg de MgO à température ambiante et on laisse sous agitation jusqu'à la fin du dégagement gazeux de $CO_2$ (durée 30 minutes).

On étête ensuite le brut réactionnel surstabilisé avec 2000 ppm de BHT sous vide à l'aide d'un évaporateur rotatif, à une température pied de 40°C maximum.

On récupère 51 g d'anhydride ayant une pureté de 95 %.

## Revendications

1. Procédé de fabrication d'un anhydride symétrique, représenté par la formule générale (I) :

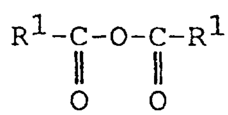

(I)

dans laquelle $R^1$ représente un groupement alkyle, alcényle, aryle, alkylaryle, aralkyle,
**caractérisé par le fait que** l'on fait réagir un anhydride mixte de formule (II) :

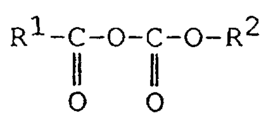

(II)

dans laquelle :

- $R^1$ est tel que défini ci-dessus ; et
- $R^2$ représente un reste alkyle en $C_1$-$C_{40}$, alcényle en $C_1$-$C_{40}$, aryle, aryl-(alkyle en $C_1$-$C_{40}$) et (alkyl en $C_1$-$C_{40}$)-aryle,

avec un acide carboxylique de formule (III) :

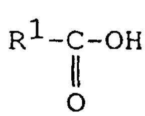

(III)

dans laquelle $R^1$ est tel que défini ci-dessus.

2. Procédé selon la revendication 1, **caractérisé par le fait que** l'on conduit la réaction avec des composés (I) et (II) dans lesquels $R^1$ représente $H_2C{=}CH-$ ou

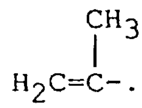

3. Procédé selon l'une des revendications 1 et 2, **caractérisé par le fait que** l'on conduit la réaction avec des composés (II) dans lesquels $R^2$ est choisi parmi méthyle, éthyle, n-propyle, isopropyle, n-butyle et isobutyle.

4. Procédé selon l'une des revendications 1 à 3, **caractérisé par le fait que** l'on introduit l'anhydride mixte (II) sous forme pure ou sous la forme du produit de réaction d'un carboxylate alcalin de formule générale (IV) :

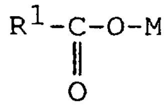 (IV)

dans laquelle :

- $R^1$ est tel que défini à la revendication 1, et
- M est un métal alcalin

avec un chloroformiate de formule générale (V) :

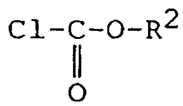 (V)

dans laquelle $R^2$ est tel que défini à la revendication 1.

5. Procédé selon la revendication 4, **caractérisé par le fait que** l'on introduit le produit de réaction du carboxylate alcalin (IV) et du chloroformiate (V), ladite réaction ayant été conduite en milieu aqueux, le carboxylate alcalin (IV) ayant pu être préparé en solution aqueuse par neutralisation de l'acide carboxylique correspondant par l'hydroxyde MOH, avant sa réaction avec le chloroformiate (V) et le produit de réaction brut ayant pu être purifié en particulier pour éliminer l'excès de chloroformiate (V).

6. Procédé selon l'une des revendications 4 et 5, **caractérisé par le fait que** le rapport molaire acide carboxylique (III)/anhydride mixte (II) est compris entre 1 et 3, de préférence entre 1 et 1,2.

7. Procédé selon l'une des revendications 4 à 6, **caractérisé par le fait que** l'on conduit la réaction à une température de -10 à 70°C, de préférence de 0 à 20°C.

8. Procédé selon l'une des revendications 4 à 7, **caractérisé par le fait que** l'on conduit la réaction entre l'anhydride mixte (II) et l'acide carboxylique (III) en présence d'un catalyseur choisi parmi les carbonates alcalins ou alcalino-terreux, les résines acides ou basiques, les substances minérales et les sels de Zn, Mn et Mg.

9. Procédé selon la revendication 8, **caractérisé par le fait que** l'on introduit le catalyseur à raison d'au moins 0,0001 mole par mole d'anhydride mixte (II), de préférence à raison de 0,001 à 0,01 mole par mole d'anhydride mixte (II).

10. Procédé selon l'une des revendications 8 et 9, **caractérisé par le fait que** le catalyseur est l'oxyde de magnésium.

**11.** Procédé selon l'une des revendications 1 à 3, **caractérisé par le fait que** l'on prépare l'anhydride mixte in situ, l'anhydride symétrique (I) étant alors obtenu directement, sans isoler l'anhydride mixte (II), par réaction d'un chloroformiate de formule (V) :

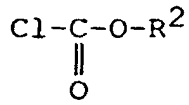

(V)

dans laquelle $R^2$ est tel que défini à la revendication 1, avec un carboxylate alcalin de formule générale (IV) :

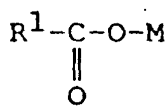

(IV)

ou un mélange carboxylate alcalin de formule (IV)/acide carboxylique (III) :

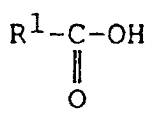

(III)

dans lesquelles :

- $R^1$ est tel que défini à la revendication 1, et
- M est un métal alcalin.

**12.** Procédé selon la revendication 11, **caractérisé par le fait que** le rapport molaire carboxylate alcalin (IV) + acide carboxylique (III)/chloroformiate (V) est compris entre 1 et 3, de préférence entre 1,5 et 1,9.

**13.** Procédé selon l'une des revendications 11 et 12, **caractérisé par le fait que** l'on conduit la réaction à une température de -10 à 70°C, de préférence de 0 à 40°C.

**14.** Procédé selon l'une des revendications 11 à 13, **caractérisé par le fait que** l'on conduit la réaction entre le carboxylate alcalin de formule générale (IV) ou le mélange carboxylate alcalin (IV) + acide carboxylique (III) et le chloroformiate de formule (V) en présence d'un catalyseur de transfert de phase, dissous ou fixé sur un support polymérique et utilisé notamment à raison de 0,001 à 0,002 mole par mole de carboxylate alcalin (IV).

**15.** Procédé selon la revendication 14, **caractérisé par le fait que** le catalyseur de transfert de phase est choisi parmi les sels d'ammonium quaternaires, les sels de phosphonium et les éthers-couronne.

**16.** Procédé selon l'une des revendications 1 à 15, **caractérisé par le fait qu'**on introduit dans le milieu réactionnel au moins un inhibiteur de polymérisation, à raison de 500 à 5000 ppm, en particulier de 500 à 1000 ppm, par rapport à l'anhydride mixte (II) ou au carboxylate alcalin (IV) de départ, dans le cas où ces derniers comportent des liaisons doubles engendrant des risques de polymérisation.

**17.** Procédé selon la revendication 16, **caractérisé par le fait que** l'inhibiteur de polymérisation est choisi parmi l'éther monométhylique de l'hydroquinone, l'hydroquinone, la phénothiazine et le ditertiobutylhydroxytoluène.

**Patentansprüche**

**1.** Verfahren zur Herstellung eines symmetrischen Anhydrids der allgemeinen Formel (I):

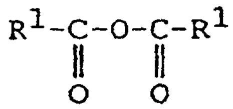

(I)

worin $R^1$ für eine Alkyl-, Alkenyl-, Aryl-, Alkylaryl- oder Aralkylgruppe steht, **dadurch gekennzeichnet, daß** man ein gemischtes Anhydrid der Formel (II):

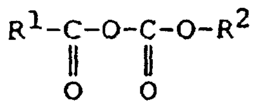

(II)

worin:

- $R^1$ die oben angegebene Bedeutung besitzt und
- $R^2$ für einen $C_1$-$C_{40}$-Alkyl-, $C_1$-$C_{40}$-Alkenyl-, Aryl-, Aryl-($C_1$-$C_{40}$-alkyl)- oder ($C_1$-$C_{40}$-Alkyl)- arylrest steht,

mit einer Carbonsäure der Formel (III):

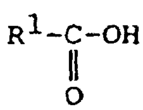

(III)

worin $R^1$ die oben angegebene Bedeutung besitzt, umsetzt.

2. Verfahren nach Anspruch 1, **dadurch gekennzeichnet, daß** man die Umsetzung mit Verbindungen (I) und (II), in denen $R^1$ für $H_2C$=CH- oder

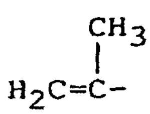

steht, durchführt.

3. Verfahren nach Anspruch 1 oder 2, **dadurch gekennzeichnet, daß** man die Umsetzung mit Verbindungen (II), in denen $R^2$ unter Methyl, Ethyl, n-Propyl, Isopropyl, n-Butyl und Isobutyl ausgewählt ist, durchführt.

4. Verfahren nach einem der Ansprüche 1 bis 3, **dadurch gekennzeichnet, daß** man das gemischte Anhydrid (II) in reiner Form oder in Form des Produkts der Umsetzung eines Alkalimetallcarboxylats der allgemeinen Formel (IV):

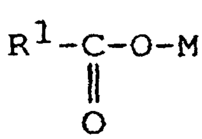

(IV)

worin:

- $R^1$ die in Anspruch 1 angegebene Bedeutung besitzt und
- M für ein Alkalimetall steht,

mit einem Chlorameisensäureester der allgemeinen Formel (V)

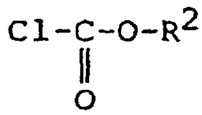

(V)

worin $R^2$ die in Anspruch 1 angegebene Bedeutung besitzt, einträgt.

5. Verfahren nach Anspruch 4, **dadurch gekennzeichnet, daß** man das Produkt der Umsetzung des Alkalimetallcarboxylats (IV) und des Chlorameisensäureesters (V) einträgt, wobei die Umsetzung in wäßrigem Medium durchgeführt worden ist, das Alkalimetallcarboxylat (IV) vor der Umsetzung mit dem Chlorameisensäureester (V) durch Neutralisation der entsprechenden Carbonsäure mit dem Hydroxid MOH in wäßriger Lösung hergestellt worden sein kann und das rohe Umsetzungsprodukt gereinigt worden sein kann, insbesondere zur Entfernung des Überschusses an Chlorameisensäureester (V).

6. Verfahren nach Anspruch 4 oder 5, **dadurch gekennzeichnet, daß** das Molverhältnis von Carbonsäure (III) zu gemischtem Anhydrid (II) zwischen 1 und 3 und vorzugsweise zwischen 1 und 1,2 liegt.

7. Verfahren nach einem der Ansprüche 4 bis 6, **dadurch gekennzeichnet, daß** man die Umsetzung bei einer Temperatur von -10 bis 70°C und vorzugsweise von 0 bis 20°C durchführt.

8. Verfahren nach einem der Ansprüche 4 bis 7, **dadurch gekennzeichnet, daß** man die Umsetzung zwischen dem gemischten Anhydrid (II) und der Carbonsäure (III) in Gegenwart eines unter Alkalioder Erdalkalimetallcarbonaten, sauren oder basischen Harzen, anorganischen Substanzen und Zn-, Mn- und Mg-Salzen ausgewählten Katalysators durchführt.

9. Verfahren nach Anspruch 8, **dadurch gekennzeichnet, daß** man den Katalysator in einer Menge von mindestens 0,0001 mol pro mol gemischtes Anhydrid (II) und vorzugsweise von 0,001 bis 0,01 mol pro mol gemischtes Anhydrid (II) einträgt.

10. Verfahren nach Anspruch 8 oder 9, **dadurch gekennzeichnet, daß** man als Katalysator Magnesiumoxid verwendet.

11. Verfahren nach einem der Ansprüche 1 bis 3, **dadurch gekennzeichnet, daß** man das gemischte Anhydrid durch Umsetzung eines Chlorameisensäureesters der Formel (V):

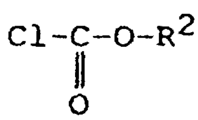

(V)

worin $R^2$ die in Anspruch 1 angegebene Bedeutung besitzt, mit einem Alkalimetallcarboxylat der allgemeinen Formel (IV):

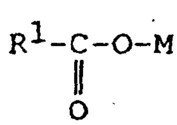

(IV)

oder einem Gemisch aus einem Alkalimetallcarboxylat der Formel (IV) und einer Carbonsäure der Formel (III):

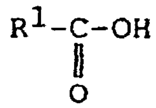

(III)

worin:

- $R^1$ die in Anspruch 1 angegebene Bedeutung besitzt und
- M für ein Alkalimetall steht, in situ herstellt, wobei man dann das symmetrische Anhydrid (I) direkt ohne Isolierung des gemischten Anhydrids (II) erhält.

**12.** Verfahren nach Anspruch 11, **dadurch gekennzeichnet, daß** das Molverhältnis von Alkalimetallcarboxylat (IV) und Carbonsäure (III) zu Chlorameisensäureester (V) zwischen 1 und 3 und vorzugsweise zwischen 1,5 und 1,9 liegt.

**13.** Verfahren nach Anspruch 11 oder 12, **dadurch gekennzeichnet, daß** man die Umsetzung bei einer Temperatur von -10 bis 70°C und vorzugsweise von 0 bis 40°C durchführt.

**14.** Verfahren nach einem der Ansprüche 11 bis 13, **dadurch gekennzeichnet, daß** man die Umsetzung zwischen dem Alkalimetallcarboxylat (IV) bzw. dem Gemisch aus Alkalimetallcarboxylat (IV) und Carbonsäure (III) und dem Chlorameisensäureester der Formel (V) in Gegenwart eines gelösten oder an einem polymeren Träger fixierten Phasentransferkatalysators durchführt, welcher insbesondere in einer Menge von 0,001 bis 0,002 mol pro mol Alkalimetallcarboxylat (IV) verwendet wird.

**15.** Verfahren nach Anspruch 14, **dadurch gekennzeichnet, daß** man den Phasentransferkatalysator unter quaternären Ammoniumsalzen, Phosphoniumsalzen und Kronenethern auswählt.

**16.** Verfahren nach einem der Ansprüche 1 bis 15, **dadurch gekennzeichnet, daß** man in dem Fall, daß das als Edukt dienende gemischte Anhydrid (II) bzw. Alkalimetallcarboxylat (IV) Polymerisationsgefahren verursachende Doppelbindungen enthalten, in das Reaktionsmedium mindestens einen Polymerisationsinhibitor in einer Menge von 500 bis 5000 ppm, insbesondere von 500 bis 1000 ppm, bezogen auf das als Edukt dienende gemischte Anhydrid (II) bzw. Alkalimetallcarboxylat (IV), einträgt.

**17.** Verfahren nach Anspruch 16, **dadurch gekennzeichnet, daß** man den Polymerisationsinhibitor unter Hydrochinonmonomethylether, Hydrochinon, Phenothiazin und Di-tert.-butylhydroxytoluol auswählt.

## Claims

**1.** Process for the manufacture of a symmetrical anhydride represented by the general formula (I):

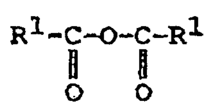

(I)

in which $R^1$ represents an alkyl, alkenyl, aryl, alkaryl or aralkyl group,
**characterized in that** a mixed anhydride of formula (II):

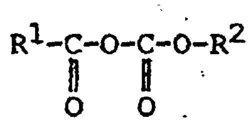 (II)

in which:

- $R^1$ is as defined above; and
- $R^2$ represents a $C_1$-$C_{40}$ alkyl, $C_1$-$C_{40}$ alkenyl, aryl, aryl-($C_1$-$C_{40}$ alkyl) and ($C_1$-$C_{40}$ alkyl) -aryl residue,

is reacted with a carboxylic acid of formula (III):

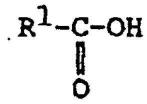 (III)

in which $R^1$ is as defined above.

2. Process according to Claim 1, **characterized in that** the reaction is carried out with compounds (I) and (II) in which $R^1$ represents $H_2C$=CH- or

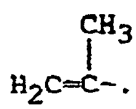

3. Process according to either of Claims 1 and 2, **characterized in that** the reaction is carried out with compounds (II) in which $R^2$ is chosen from methyl, ethyl, n-propyl, isopropyl, n-butyl and isobutyl.

4. Process according to one of Claims 1 to 3, **characterized in that** the mixed anhydride (II) is introduced in pure form or in the form of the product of the reaction of an alkali metal carboxylate of general formula (IV):

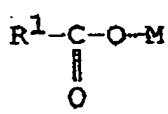 (IV)

in which:

- $R^1$ is as defined in Claim 1, and
- M is an alkali metal

with a chloroformate of general formula (V):

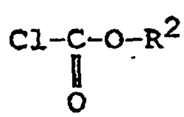 (V)

in which $R^2$ is as defined in Claim 1.

**5.** Process according to Claim 4, **characterized in that** there is introduced the product of the reaction of the alkali metal carboxylate (IV) and the chloroformate (V), said reaction having been performed in an aqueous medium, the alkali metal carboxylate (IV) having possibly been prepared in aqueous solution by neutralizing the corresponding carboxylic acid with the hydroxide MOH, before its reaction with the chloroformate (V), and the crude reaction product having possibly been purified in particular in order to remove the excess of chloroformate (V).

**6.** Process according to either of Claims 4 and 5, **characterized in that** the carboxylic acid (III)/mixed anhydride (II) molar ratio is between 1 and 3, preferably between 1 and 1.2.

**7.** Process according to one of Claims 4 to 6, **characterized in that** the reaction is carried out at a temperature of -10 to 70°C, preferably 0 to 20°C.

**8.** Process according to one of Claims 4 to 7, **characterized in that** the reaction between the mixed anhydride (II) and carboxylic acid (III) is carried out in the presence of a catalyst chosen from alkali or alkaline-earth metal carbonates, acidic or basic resins, inorganic substances and salts of Zn, Mn and Mg.

**9.** Process according to Claim 8, **characterized in that** the catalyst is introduced in an amount of at least 0.0001 mol per mol of mixed anhydride (II), preferably in an amount of 0.001 to 0.01 mol per mol of mixed anhydride (II).

**10.** Process according to either of Claims 8 and 9, **characterized in that** the catalyst is magnesium oxide.

**11.** Process according to one of Claims 1 to 3, **characterized in that** the mixed anhydride is prepared in situ, the symmetrical anhydride (I) then being obtained directly, without isolating the mixed anhydride (II), by reacting a chloroformate of formula (V):

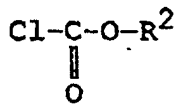 (V)

in which $R^2$ is as defined in Claim 1,
with an alkali metal carboxylate of general formula (IV):

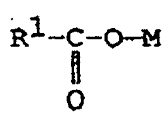 (IV)

or an alkali metal carboxylate of formula (IV)/carboxylic acid (III) mixture:

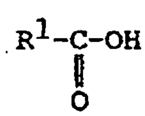 (III)

in which:

- $R^1$ is as defined in Claim 1, and
- M is an alkali metal.

**12.** Process according to Claim 11, **characterized in that** the alkali metal carboxylate (IV) + carboxylic acid (III)/chloroformate (V) molar ratio is between 1 and 3, preferably between 1.5 and 1.9.

**13.** Process according to either of Claims 11 and 12, **characterized in that** the reaction is carried out at a temperature of -10 to 70°C, preferably 0 to 40°C.

14. Process according to one of Claims 11 to 13, **characterized in that** the reaction between the alkali metal carboxylate of the general formula (IV) or the alkali metal carboxylate (IV) + carboxylic acid (III) mixture and the chloroformate of formula (V) is advantageously carried out in the presence of a phase transfer catalyst, dissolved or attached to a polymeric carrier and used in particular in an amount of 0.001 to 0.002 mol per mol of alkali metal carboxylate (IV).

15. Process according to Claim 14, **characterized in that** the phase transfer catalyst is chosen from quaternary ammonium salts, phosphonium salts and crown ethers.

16. Process according to one of Claims 1 to 15, **characterized in that** there is introduced into the reaction medium at least one polymerization inhibitor, in an amount of 500 to 5000 ppm, in particular 500 to 1000 ppm, compared with the starting mixed anhydride (II) or alkali metal carboxylate (IV) when the latter contain double bonds causing risks of polymerization.

17. Process according to Claim 16, **characterized in that** the polymerization inhibitor is chosen from hydroquinone monomethyl ether, hydroquinone, phenothiazine and di-tert-butyl hydroxytoluene.